# EUROPEAN PATENT APPLICATION

(11) **EP 1 319 414 A1**
(43) Date of publication of application: **18.06.2003**
(21) Application number: 02021370.8
(22) Date of filing: 24.09.2002
(51) Int. Cl.: A61L 15/22, A61L 15/60

(54) **Liquid absorbing thermoplastic materials and the utilization thereof in absorbent articles**

(30) Priority: 11.12.2001 EP 01129529
(71) Applicant: The Procter & Gamble Company, Cincinnati, Ohio 45202 (US)
(72) Inventor: Bonfanti, Lidia, 66020 Sambuceto di S. Giovanni Chietino (IT); Corzani, Italo, 66100 Chieti (IT); Gonzales, Denis Alfred, 65100 Pescara (IT); Spina, Enrico, 65100 Pescara (IT)
(74) Representative: Veronese, Pancrazio

(57) **Abstract**

The present invention relates to improved liquid absorbing thermoplastic materials useful for example as absorbent materials for disposable absorbent articles. The liquid absorbing thermoplastic materials comprise A) thermoplastic compositions comprising thermoplastic polymers and suitable compatible plasticisers and B) superabsorbent particles dispersed in the thermoplastic compositions. Such liquid absorbing thermoplastic material, preferably in form of hot melt compositions, can be utilised in the absorbent cores of disposable absorbent articles, such as sanitary napkins, panty liners, disposable diapers, nursing pads and the like for completely or partially substituting said absorbent cores.

## Description

### Field of the Invention

The present invention relates to liquid absorbing thermoplastic materials. The liquid absorbing thermoplastic materials comprise A) a thermoplastic composition comprising thermoplastic polymers and suitable compatible plasticisers and B) superabsorbent particles dispersed in the thermoplastic composition. Such liquid absorbing thermoplastic materials can be utilized in a number of end uses, for example in the absorbent core of disposable absorbent articles, such as sanitary napkins, panty liners, disposable diapers, nursing pads and the like for completely or partially substituting said absorbent core.

### Background of the Invention

In general the absorption and retention of aqueous liquids, particularly body fluids such as urine, menses, etc., are accomplished by use of absorbent articles containing absorbent materials. Such articles include disposable diapers, incontinence pads, sanitary napkins, tampons, wound dressings, nursing pads, and the like. Generally, the most used absorbent materials are cellulose materials (preferably, defiberised wood pulp) and superabsorbent materials. In particular, when referring to disposable diapers or sanitary napkins and the like presently available in the market, the cellulose materials are in the form of bat or sheet, typically further containing particulate absorbent materials, usually referred to in the art as superabsorbents or hydrogelling materials, which allow to manufacture thin but very absorbent core structures. Supported or unsupported films made entirely from superabsorbent polymers have been suggested as absorbent core for disposable absorbent articles but they are generally stiff and frequently break apart especially when in dry state. In addition to these solutions, in the art there are different suggestions of water-insoluble polymeric thermoplastic sheet materials containing superabsorbent material. For example, US 4318408 describes an aqueous-fluid-absorbent non-disintegrative product which comprises a water-insoluble substantially non-swelling matrix of an elastomeric polymer having at least partially embedded therein particles of a water-insoluble but water-swellable organic polymeric absorbent material. Based on the type of elastomeric polymers described, the matrix has no absorbent properties. Similarly, EP 1013291 describes a hot melt adhesive containing a superabsorbent polymer. WO 99/57201 illustrates compositions comprising a thermoplastic component and a superabsorbent polymer, said compositions in form of a film layer or applied to a disposable absorbent article with various hot melt adhesive application techniques. While this last solution provides an improvement over the above art in the sense of a more complete utilization of the absorbent capacity of the superabsorbent particles, this improvement is achieved by the use of a non absorbent matrix which generally decreases its integrity when wet in order to achieve a free swelling of the superabsorbent particles upon liquid absorption.

In addition, the art fails to recognize that such structures and the similar ones also described in the prior art do not work well especially when used in a breathable absorbent product, as it will be explained hereinbelow in the particular context of disposable absorbent articles.

As a matter of fact many known disposable absorbent articles utilize absorbent materials located between a liquid pervious topsheet and a vapor and liquid impermeable backsheet. Such backsheets are effective in preventing the migration of body fluids from the absorbent materials to the garments of a wearer. However, the use of liquid and vapor impermeable backsheets can result in a high degree of humidity within the absorbent product during its use, which causes discomfort to the user and may result in a temperature increase of human skin and in relatively high skin hydration levels. In addition, the moist environment within the disposable absorbent product may also promote the growth of microorganisms.

In order to decrease the humidity level within disposable absorbent articles during their use, particularly disposable diapers and sanitary napkins, porous polymeric films have been employed as breathable backsheets for such absorbent products. These breathable films typically present micropores and provide a compromise between desired levels of liquid impermeability and vapour permeability. However, in certain circumstances of use of such absorbent articles, microporous films can exhibit excessive leakage of liquids from the article, thus leading to soiling of the outer clothes of the user. This problem can be solved by the use of monolithic films as backsheets, i.e. by the use of continuous breathable films which do not allow the flow of moisture vapour through open pores or apertures in the material but which transmit water vapour by absorbing the water vapour on the side of the film where the partial vapour pressure is higher (e.g. inside the absorbent article) and desorbing the water vapour to the side of the film where the same partial pressure is lower (e.g. outside the absorbent article). In any case, while the use of breathable films as backsheet improves the comfort for the user especially during the initial phases of wearing of the absorbent article, the performance of such absorbent articles can be still improved. More in general, breathability of an absorbent structure both in dry and in wet state can provide an advantage also in fields of application different from disposable absorbent articles.

On one hand in fact, prior art composite absorbent structures comprising a thermoplastic matrix (e.g. a hot melt adhesive), and superabsorbent particles, have poor liquid handling/absorption capability, or lack integrity in the wet state, and in any case are not breathable per se, and may be not fully compatible (e.g. chemically) with other materials with which the composite absorbent structure may come in contact, for example, in a disposable absorbent article.

On the other hand, in the specific field of disposable absorbent articles, when prior art traditional absorbent cores based on absorbent cellulose materials become loaded with body fluids, the wet absorbent core loses most of its permeability for water vapour and, consequently, the core can impede the escape of water vapour from the skin of the user. Therefore, while such breathable absorbent structures, typically comprised in breathable absorbent articles, have a high level of breathability when dry or loaded with small quantities of body fluids, their level of breathability, when wet in their intended use, can decrease to a level not sufficient for assuring adequate comfort to the user.

Therefore there is a need for an absorbent material, to be for example employed in the absorbent cores of disposable absorbent articles, which, in addition to having all the characteristics for its intended use, offers improved mechanical and absorbent properties and, preferably, is also breathable even when loaded with a substantial amount of liquid, as experienced during use of such absorbent articles.

It is therefore an object of the present invention to provide improved liquid absorbing thermoplastic materials, which can be used as absorbent material e.g. for absorbent cores of disposable absorbent articles.

Particularly, it is an object of the present invention to provide liquid absorbing thermoplastic materials, which can be used for example as absorbent material for absorbent cores of disposable absorbent articles, having improved mechanical properties.

It is a further object of the present invention to provide liquid absorbing thermoplastic materials, which can be used for example as absorbent material for absorbent corès of disposable absorbent articles, having breathability properties.

Other objects and more specific properties of the absorbent material according to the present invention will be clear after reading the following description of the invention.

### Summary of the Invention

The present invention provides a liquid absorbing thermoplastic material, which can be used for example as an absorbent material for disposable absorbent articles, comprising a matrix of a thermoplastic polymeric composition having particles of water-insoluble water-swellable absorbent material dispersed therein, wherein the thermoplastic polymeric composition comprises:
a thermoplastic polymer or a mixture of thermoplastic polymers, and
a suitable compatible plasticiser or a blend of suitable compatible plasticisers,
The thermoplastic polymeric composition has a water absorption greater than 30%, preferably greater than 40%, more preferably greater than 60%, most preferably greater than 90%, when the water absorption is measured according to the Water Absorption Test described herein.

An absorbent article comprising a liquid permeable topsheet, a liquid impermeable backsheet and an absorbent core therebetween, wherein the absorbent core is made of or comprises a liquid absorbing thermoplastic material according to the present invention is also provided according to the present invention. In a preferred embodiment said absorbent article comprises as the backsheet a breathable backsheet.

### DESCRIPTION OF THE INVENTION

By "liquid" as herein used is meant water based fluids or liquids such as urine, menses, serum, blood, sweat, mucous as well as other aqueous solutions generally defined as body fluids, but it is not intended to exclude other water based fluids.

The liquid absorbing thermoplastic materials (i.e. the absorbent materials), according to the present invention, comprise:
A) a matrix of a thermoplastic polymeric composition comprising thermoplastic polymers and suitable compatible plasticisers, and
B) particles of water-insoluble, water-swellable absorbent material, i.e., superabsorbent particles, dispersed into the matrix of thermoplastic composition.

The absorbent materials according to the present invention will be now described with reference to the above components A and B.

### A) The matrix of thermoplastic polymeric composition.

According to the present invention the matrix of a thermoplastic polymeric composition comprises:
a thermoplastic polymer or a mixture of thermoplastic polymers, and
a suitable compatible plasticiser or a blend of suitable compatible plasticisers,
and has a water absorption greater than 30%, preferably greater than 40%, more preferably greater than 60%, most preferably greater than 90%, when said water absorption is measured according to the Water Absorption Test described hereinbelow.

As stated in the background of the invention, WO 99/57201 discloses compositions comprising a thermoplastic component and superabsorbent polymer particles in form of a film layer or applied to a disposable absorbent article with various hot melt adhesive application techniques. According to this patent application the thermoplastic composition should be sufficiently polar and of low cohesive strength in order to allow the superabsorbent particle swelling upon liquid absorption. It is believed that liquid acquisition is likely triggered by the swelling of the superabsorbent particles initially reached by the liquid, which in turn could cause the partial rupturing of the thermoplastic matrix and the creation of paths for further liquid migration within the thermoplastic matrix. This means that the absorbing hot melt, according to the above patent application, has rather weak mechanical strength, particularly in wet state, since the swelling capability of a superabsorbent particle is achieved at the expense of the cohesive strength of the thermoplastic composition containing the particle.

Contrary to the above mechanism, the inventors have surprisingly discovered that if the matrix of thermoplastic polymeric composition according to the present invention has a water absorption, as measured according to the Water Absorption Test described herein, greater than 30%, preferably greater than 40%, more preferably greater than 60%, most preferably greater than 90%, good absorption characteristics can be obtained with a good strength of the matrix, and therefore of the absorbent material, also in the wet state. While not wishing to be bound by a theory, the inventors believe that the intrinsic absorbency of the matrix allows the liquid to be acquired and diffused within the matrix thus permitting the liquid contact with the superabsorbent particles contained in the matrix and their swelling, without the necessity'of having a matrix of low cohesive strength but with a matrix which remains substantially intact and having sufficient strength upon liquid absorption.

It should be also appreciated that the intrinsic absorbency of the matrix allows for a more effective diffusion of the liquid within the matrix and, consequently, for a better spreading of the liquid which can reach a greater number of superabsorbent particles which in turn give rise to a better utilization of the absorbent material of the present invention compared to those of the prior art.

In addition to the above selection criteria for the matrix of thermoplastic polymeric composition, the inventors have found that the thermoplastic polymer or polymers to be used in the matrix according to the present invention shall preferably have a water absorption greater than 10%, preferably greater than 20%, more preferably greater than 30%, as measured according to the Water Absorption Test described herein.

As stated in the background of the invention, thermoplastic films which provide a liquid barrier in addition to providing moisture vapour permeability are known in the art. Particularly suitable are hydrophilic continuous films that do not allow the flow of moisture vapour through open pores or apertures in the material, but do transfer substantial amounts of moisture vapour through the film by absorbing water on one side of the film where the moisture vapour concentration is higher, and desorbing or evaporating it on the opposite side of the film where the moisture vapour concentration is lower. Such films are typically formed from a thermoplastic polymeric composition comprising a thermoplastic hydrophilic polymer, or a blend of thermoplastic hydrophilic polymers. Thermoplastic hydrophilic polymeric compositions having the above described characteristics are also known in the art as "monolithic compositions", and the moisture vapour permeable, liquid impermeable layers or films made therefrom are known as "monolithic layers" or "monolithic films". They are breathable, i.e. they are impermeable but allow the passage of water vapour.

Thermoplastic polymers to be used in the matrix according to the present invention can be preferably selected, following the above criteria of water absorption for the thermoplastic polymeric composition and, preferably, for the thermoplastic polymer or polymers, among monolithic breathable polymers, in order to provide liquid absorbing thermoplastic materials which are preferably also breathable.

Suitable thermoplastic polymers comprised in the thermoplastic composition for the matrix of thermoplastic polymeric composition according to the present invention can be selected following the above criteria on water absorption, e.g. from those described in the patent applications WO 99/64077 and WO 99/64505, and include polyurethanes, poly-ether-amides block copolymers, polyethylene-acrylic acid and polyethylene-methacrylic acid copolymers, polyethylene oxide and its copolymers, ethylene acrylic esters and ethylene methacrylic esters copolymers, poly lactide and copolymers, polyamides, polyesters and copolyesters, polyester block copolymers, sulfonated polyesters, poly-ether-ester block copolymers, poly-ether-ester-amide block copolymers, polyacrylates, polyacrylic acids and derivatives, ionomers, polyethylene-vinyl acetate with a vinyl acetate content of at least 28% by weight, polyvinyl alcohol and its copolymers, polyvinyl ethers and their copolymers, poly-2-ethyl-oxazoline and derivatives, polyvinyl pyrrolidone and its copolymers, thermoplastic cellulose derivatives, poly-caprolactone and copolymers, poly glycolide, polyglycolic acid and copolymers, polylactic acid and copolymers, polyureas, or mixtures thereof. Said polymers preferably have a water absorption greater than 10%, preferably greater than 20%, more preferably greater than 30%, as measured according to the Water Absorption Test described herein.

Particularly suitable preferred thermoplastic breathable polymers are selected from thermoplastic poly-ether-amide block copolymers (e.g. Pebax™), thermoplastic poly-ether-ester-amide block copolymers, thermoplastic polyester block copolymers (e.g. Hytrel™), thermoplastic polyurethanes (e.g. Estane™), or mixtures thereof, provided that the above selection criteria in terms of water absorption are met.

As it is known, such thermoplastic polymers or mixture of polymers can be typically highly viscous in the molten state and they can be processed in a film or a layer only by an extrusion process involving a high power screw extruder.

As disclosed in the above mentioned patent applications WO 99/64077 and WO 99/64505, the viscosity of the thermoplastic compositions comprised in the thermoplastic matrix of the present invention and comprising the thermoplastic polymers or mixture of polymers can be preferably adjusted by including in the thermoplastic composition a suitable plasticiser, or a blend of suitable plasticisers, that is compatible with the thermoplastic polymers and that lowers the viscosity of the thermoplastic polymer or mixture of polymers in the molten state.

By lowering the viscosity of the thermoplastic compositions they are more easily processable. For example, said thermoplastic compositions can allow for a film or layer to be e.g. formed using apparatuses known in the art for processing low viscosity hot melt compositions in a layer having a required thickness, while also keeping the preferred advantageous characteristics of the above thermoplastic polymers in providing hydrophilic continuous moisture vapour permeable, liquid impermeable layers or films (i.e. monolithic films) also bearing (as explained hereinafter) uniformly dispersed water-insoluble water-swellable absorbent material particles.

Suitable compatible plasticisers comprised in the thermoplastic hydrophilic polymeric composition according to this preferred embodiment of the present invention include citric acid esters, tartaric acid esters, glycerol and its esters, sucrose esters, adipates, sebacates, sorbitol, epoxidized vegetal oils, polymerised vegetal oils, polyols, phthalates, liquid polyesters, glycolates, p-toluene sulfonamide and derivatives, glycols and polyglycols and their derivatives, sorbitan esters, phosphates, monocarboxylic fatty acids (C₈-C₂₂) and their derivatives, and mixtures thereof.

According to a particularly preferred embodiment of the present invention particularly preferred plasticisers to be used in the matrix of thermoplastic polymeric composition are hydrophilic plasticisers such as acids, esters, amides, alcohols, polyalcohols, or mixtures thereof, among which even more preferred are citric acid esters, tartaric acid esters, glycerol and its esters, sorbitol, glycolates, and mixtures thereof, as disclosed in our application WO 99/64505. Said particularly preferred hydrophilic plasticisers have a particularly high polar character and provide the further advantage that they do not impair, and possibly can even enhance, the moisture vapour permeability of the resulting layer or film formed from the preferred thermoplastic hydrophilic polymeric composition of the present invention comprising said preferred plasticiser or blend of plasticisers, when compared to a corresponding film or layer formed from a thermoplastic hydrophilic polymeric composition comprising the same components, but without the plasticiser or plasticisers.

These particularly preferred hydrophilic plasticiser or blend of hydrophilic plasticisers can of course also adjust the viscosity of the thermoplastic polymeric composition according to a preferred embodiment of the present invention to the preferred values in order to make it processable by coating said thermoplastic composition onto a substrate in a layer or film having a desired thickness.

Plasticisers selected among those described in our copending European application EP 00121585.4, filed on 2 October 2000 and entitled "Improved thermoplastic hydrophilic polymeric compositions for moisture vapour permeable structures", can also be used in the thermoplastic hydrophilic polymeric compositions of the present invention. Said plasticisers can be selected from the group consisting of esters of phosphoric acid; esters of benzoic, phthalic and trimellitic acids; esters of polycarboxylic oxy-acids; sulphonamides and their derivatives such as sulphonamide-formaldehyde resins; sulfones; esters of polyvalent alcohols; lactides; glycolides; lactones; lactams. Said plasticisers are capable of also providing the thermoplastic composition with a certain degree of tackiness.

In one aspect of the invention it should be noted that by selecting the components of the matrix of the thermoplastic polymeric composition in the liquid absorbing thermoplastic materials of the present invention as explained above a further benefit may be attained: i.e. owing to the breathability of the matrix, also the liquid absorbing thermoplastic material or absorbent material according to the present invention is breathable. Due to the intrinsic breathability of the matrix in dry and wet state, the absorbent material according to the present invention is breathable not only in dry state but also in the wet one. It is believed that the wet breathability of the absorbent material is due to the fact that the breathable matrix remains substantially intact after wetting while allowing the superabsorbent particles to swell but maintaining them separated and fixed. In other words, the matrix which is impermeable to liquids but permeable to water vapour by remaining substantially intact after wetting, maintains its breathability characteristics even in the wet state, thus conferring effective breathability to the absorbent material according to the present invention.

Preferred breathability values, expressed as moisture vapour transmission rate (MWTR) and measured according to the Moisture Vapour Transmission Test described herein, for the liquid absorbing thermoplastic material of the present invention, when formed into continuous films or layers, are of at least 300 g/m²·24h, more preferably of at least 500 g/m²·24h, even more preferably of at least 600 g/m²·24h, most preferably of at least 1000 g/m²·24h, with a thickness of said layer or film of at least 20 µm.

According to a further preferred embodiment of the present invention, the inventors have further found that for avoiding migration of the plasticizer or blend of plasticizers from the matrix of thermoplastic polymeric composition it is preferable that the molecular weight (MW) of the selected plasticiser or plasticisers be greater than 300, preferably greater than 1000, and more preferably greater than 3000. Plasticisers having a MW of at least 6000 work particularly well.

This is particularly important when the liquid absorbing thermoplastic material according to the present invention is used in disposable absorbent articles. As a matter of fact these articles are assembled by and contain conventional hydrophobic hot melt adhesive which can interact with the absorbent material of the present invention and particularly with its hydrophilic plasticizer giving rise to a degradation of the adhesive characteristics of the conventional hot melt adhesive. For example, a sanitary napkin or a panty liner is generally made of a liquid permeable topsheet, of a liquid impermeable backsheet, and of an absorbent core therebetween. Generally the outside of the backsheet is provided with stripes of conventional hot melt pressure sensitive adhesive for fastening the absorbent article to the user's panty. If said absorbent core is substituted in total or in part by a core made of the absorbing material according to the present invention, for example coated onto the inner surface of the backsheet, the inventors have surprisingly found that if the hydrophilic plasticiser or the blend of hydrophilic plasticisers are selected from those having a molecular weight (MW) greater than 300, preferably greater than 1000, more preferably greater than 3000, there is no interference with the adhesive for fastening the absorbent article, i.e. no peel reduction of said adhesive is experienced, while using hydrophilic plasticizers of lower molecular weight a significant reduction of adhesive peel is experienced, presumably due to plasticiser migration out of the liquid absorbing thermoplastic material.

Preferably, plasticisers having the preferred molecular weight as explained above can be selected from the group consisting of glycerol esters, sucrose esters, sorbitol, epoxidized vegetal oils, polymerised vegetal oils, polyalcohols, polyols, liquid polyesters, p-toluene sulfonamide and derivatives, polyglycols and their derivatives, monocarboxylic fatty acids (C₈-C₂₂) and their derivatives, and mixtures thereof, wherein polyglycols and their derivatives are particularly preferred (i.e. polyethylene glycols and polypropylene glycols).

Of course, the preferred plasticisers disclosed above can also be used in thermoplastic compositions alone, i.e. without the addition of any water insoluble water swellable absorbent material. The reduced tendency to migrate from the thermoplastic composition achieved by these preferred plasticisers can in fact find utility also besides the liquid absorbing thermoplastic compositions of the present invention.

Preferably, the matrix of thermoplastic polymeric composition comprised in the liquid absorbing thermoplastic material according to the present invention comprises from 10% to 80%, preferably from 15% to 65%, and more preferably from 20% to 40% by weight of the thermoplastic polymeric composition, of the thermoplastic polymer or mixture of polymers,-and from 20% to 90%, preferably from 35% to 85%, and more preferably from 60% to 80% by weight of the thermoplastic composition, of the suitable compatible plasticiser or blend of suitable compatible plasticisers.

### B) The water-insoluble but water-swellable absorbent polymer.

Water-insoluble but water-swellable absorbent materials or absorbent gelling materials are usually referred to as "hydrogels", "superabsorbent", "absorbent gelling" materials. Absorbent gelling materials are those materials that, upon contact with aqueous fluids, especially aqueous body fluids, imbibes such fluids and thus form hydrogels. These absorbent gelling materials are typically capable of absorbing large quantities of aqueous body fluids, and are further capable of retaining such absorbed fluids under moderate pressures. These absorbent gelling materials are typically in the form of discrete particles. Particles can have different size, shape and morphology, and, although not preferably, can be in form of fibres.

Any commercially available superabsorbent material in particle form is suitable for the present invention.

Suitable absorbent gelling materials for use herein will most often comprise a substantially water-insoluble, slightly crosslinked, partially or fully neutralized, polymeric gelling material. This material forms a hydrogel upon contact with water. Such polymer materials can be prepared from polymerizable, unsaturated, acid-containing monomers. Suitable unsaturated acidic monomers for use in preparing the polymeric absorbent gelling material used in this invention include those listed in U.S. Patent 4,654,039 and reissued as RE 32,649. Preferred monomers include acrylic acid, methacrylic acid, and 2-acrylamido-2-methyl propane sulfonic acid. Acrylic acid itself is especially preferred for preparation of the polymeric gelling material. The polymeric component formed from the unsaturated, acid-containing monomers can be grafted onto other types of polymer moieties such as starch or cellulose. Polyacrylate grafted starch materials of this type are especially preferred. Preferred polymeric absorbent gelling materials that can be prepared from conventional types of monomers include hydrolyzed acrylonitrile grafted starch, polyacrylate grafted starch, polyacrylates, maleic anhydride-based copolymers and combinations thereof. Especially preferred are the polyacrylates and polyacrylate grafted starch, but other absorbent substances in particle form such as inorganic materials (MgSO4, CaCl2, silicas, zeolites, etc.) or absorbing polymers (i.e. chitin derivatives such as, for example, chitosan) can be used alone but, preferably, blended with the above superabsorbent polymers.

Depending on the selected process utilized for applying the liquid absorbing thermoplastic material of the present invention it can be preferable that the particle size of the superabsorbent material is below 150 micrometers, and more preferably below 50 micrometers. "Particle size" as used herein means the weighted average of the smallest dimension of the individual particles.

It is preferable hat the superabsorbent material is present in an amount from 10% to 90%, preferably from 15% to 70%, more preferably from 20% to 60% by weight of the liquid absorbing thermoplastic material according to the present invention.

The chosen superabsorbent or mixture of chosen superabsorbents in particle form are blended with the thermoplastic composition in any known manner to provide the liquid absorbing thermoplastic material according to the present invention. For example, by first melting the thermoplastic composition and then by adding and mixing the required amount of superabsorbent particles.

### Other ingredients

The liquid absorbing thermoplastic materials of the present invention may in addition comprise additional optional components to further improve the processibility and also the mechanical characteristics as well as other characteristics as tackiness, resistance to ageing by light and oxygen, visual appearance etc.

Such optional components include tackifying resins or blends of tackifying resins having a softening point of 125°C or less. Preferably they have a high MW and they are solid at room temperature. Suitable resins, which may be present by up to 50%, preferably less than 20% by weight of the matrix of the thermoplastic polymeric composition according to the present invention, may be selected from rosins and rosin esters, hydrocarbon resins, aliphatic resins, terpene and terpene-phenolic resins, aromatic resins, synthetic C₅ resins, mixtures of synthetic C₅-C₉ resins, and mixtures thereof.

Other optional components of said liquid absorbing thermoplastic materials include anti-oxidants, anti-ultraviolets, water scavengers, hydrolytic stabilizers, pigments, dyes, antibacterials, odour adsorbing materials, perfumes, pharmaceuticals, and mixtures thereof, which may be present within the compositions at a level of up to 10% by weight of the composition.

### PROCESS

Preferably the liquid absorbing thermoplastic materials according to the present invention are formulated to have hot melt characteristics so that they can be applied utilizing any know method used for applying hot melt adhesives.

A possible method for forming the absorbent material of the present invention having preferred hot melt characteristics is by coating the liquid absorbing thermoplastic material according to the present invention onto a substrate acting as a support layer as described in PCT application WO 96/25902.

At least at the coating temperature, since the liquid absorbing thermoplastic material comprises thermoplastic compositions, it can exhibit adhesive properties on a supportive substrate in order to form a composite structure such that no additional adhesive is required to achieve a permanent attachment between the absorbent material and the substrate. The liquid absorbing thermoplastic compositions of the present invention can therefore be also formulated and used as an absorbent adhesive. In some applications it may be also desirable that the absorbent material remains tacky at any temperature. In this case the absorbent material should be formulated so to have the typical characteristics of a pressure sensitive adhesive.

However, while hot melt techniques are preferred, any other known method for processing thermoplastic compositions can be used for processing the absorbent material according to the present invention.

In addition, the absorbent material according to the present invention may be applied utilizing any solvent technique, as it is known in the art.

### APPLICATIONS

As stated hereinbefore, the liquid absorbing thermoplastic materials according to the present invention are absorbent materials. Therefore, they can be utilised in substituting totally or partially conventional absorbent materials in all applications where absorbent materials are used.

While it is not intended to limit the use of the absorbent materials according to the present invention to any useful utilization, at present its preferred use is in the field of disposable absorbent articles, such as sanitary napkins, panty liners, incontinence devices, nursing pads, baby diapers, and the like.

Typically such products comprise a liquid pervious topsheet, a liquid impervious backsheet and an absorbent core therebetween. Consequently, the absorbent material according to the present invention may be used for totally or partially substituting said absorbent core, for example with the liquid absorbing thermoplastic material coated onto a substrate, e.g. the backsheet..

A particularly preferred application is as breathable absorbent material for cores of breathable sanitary napkins and panty liners. As mentioned in the background of the invention, when the conventional absorbent core of the absorbent article becomes loaded with body fluids, it loses most of its permeability for water vapour and, consequently, it may impede the escape of water vapour from the skin of the user. Therefore, while such breathable absorbent articles have a high level of breathability when dry or loaded with small quantities of body fluids, their level of breathability, when wet in their intended use, may decrease to a level not sufficient for assuring adequate comfort to the user.

By substituting totally or partially the conventional absorbent material of the absorbent cores of said absorbent articles with the preferably breathable absorbent material according to the present invention, the absorbent articles will be breathable even when loaded with substantially amount of liquids as experienced during use of such absorbent articles.

In any case the improved absorbent material according to the present invention due to its other improved characteristics, namely improved mechanical properties, can be employed advantageously also in non breathable absorbent articles.

Besides disposable absorbent articles, the liquid absorbing thermoplastic compositions of the present invention can also find utility in a variety of end-uses, such as for example in cable applications, in packaging e.g. of food and drugs, in building and agricultural applications, in order to provide liquid and moisture absorption.

While the absorption characteristics (e.g. the liquid absorption in terms of grams of liquid absorbed for each gram of absorbent material) of the liquid absorbing thermoplastic material according to the present invention can be adjusted by varying the percentage and the type of superabsorbent material contained in the thermoplastic matrix according to the invention, preferably the liquid absorbing thermoplastic material of the present invention has a liquid absorption of at least 5 g/g of a saline solution, such as a NaCI 0.9% water solution, preferably of at least 7 g/g, more preferably of at least 10 g/g.

The liquid absorption of the liquid absorbing thermoplastic materials of the present invention can be evaluated according to the Water Absorption Test described herein, made on the material in form of a film sample 76.2 mm long by 25.4 mm wide by 0.2 mm thick, and using the saline solution mentioned above instead of distilled water.

As stated hereinbefore, improved absorption characteristics of the liquid absorbing thermoplastic material (i.e. the absorbent material) according to the present invention can be obtained with a good strength of the absorbent material, also in the wet state.

The intrinsic absorbency of the thermoplastic matrix according to the present invention allows for a more effective diffusion of the liquid within the matrix and, consequently, for a better spreading of the liquid which can reach a greater number of superabsorbent particles which in turn give rise to a better utilization of the absorbent material of the present invention compared to those of the prior art.

The intrinsic absorbency of the matrix allows the liquid to be acquired and diffused within the matrix thus permitting the liquid contact with the superabsorbent particles contained in the matrix and their swelling, without the necessity of having a matrix of low cohesive strength but with a matrix which remains substantially intact and having sufficient strength upon liquid absorption.

In one embodiment the liquid absorbing thermoplastic materials according to the present invention have a tensile strength in wet state which is at least 20%, preferably at least 40%, and more preferably 60% of the tensile strength of said material in dry state. Said tensile strengths are evaluated according to the Tensile Strength Test described herein.

It should be appreciated that by selecting a thermoplastic composition, in the liquid absorbing thermoplastic material of the present invention, having a higher value of water absorption, the absorbent material of the present invention will have better liquid absorption/handling characteristics, but with a slightly lesser tensile strength in wet state; however, the absorbent material according to the present invention will remain substantially intact and have sufficient tensile strength for its intended use, also upon liquid absorption.

### EXAMPLES ACCORDING TO THE INVENTION

### Example 1

A thermoplastic polyether-amide block copolymer available from Atofina (France) under the trade name Pebax MV 3000 is compounded with polyethylene glycol PEG 400 (plasticiser, MW about 400), Sodium Dodecyl Sulphate (SDS), both available from Aldrich Co., and Irganox B 225 (anti oxidant agent) available from Ciba-Geigy.

The formulation in percent by weight has the following composition, and constitutes the thermoplastic polymeric composition:

| | |
|---|---|
| 28.6% | Pebax MV 3000 |
| 68.6% | PEG 400 |
| 1.4% | SDS |
| 1.4% | Irganox B 225 |

The thermoplastic polymeric composition has a water absorption of 43%, while Pebax MV 3000 has a water absorption of 34%, both values measured according to the Water Absorption Test described herein. The thermoplastic composition is formed into a film to be used in the Water absorption Test by melt coating the thermoplastic composition at a temperature of 180°C onto a release paper to obtain a film having the prescribed thickness of 200 µm. After cooling at room temperature the film is separated from the release paper.

A superabsorbent material in particle form available from Stockhausen GmbH & Co. KG under the code Z3049 is added to the thermoplastic polymeric composition while maintained at a temperature of 180°C and uniformly dispersed, in an amount corresponding to 42.9% by weight of the thermoplastic composition. The liquid absorbing thermoplastic material (Composition 1) has the following final composition by weight:

| | |
|---|---|
| 20% | Pebax MV 3000 |
| 48% | PEG 400 |
| 30% | Z3049 |
| 1% | SDS |
| 1% | Irganox B 225 |

### Example 2

A thermoplastic polyether-ester block copolymer available from Du Pont (USA) under the trade name Hytrel 8171 is compounded with polyethylene glycol PEG 400 (plasticiser, MW about 400), polyethylene glycol PEG 1500 (plasticiser, MW about 1500), both available from Aldrich Co., and Irganox B 225 (anti oxidant agent) available from Ciba-Geigy.

The formulation in percent by weight has the following composition, and constitutes the thermoplastic polymeric composition:

| | |
|---|---|
| 28.6% | Hytrel 8171 |
| 21.4% | PEG 400 |
| 28.6% | PEG 1500 |
| 1.4% | Irganox B 225 |

The thermoplastic polymeric composition has a water absorption of 96%, while Hytrel 8171 has a water absorption of 68%, both values measured according to the Water Absorption Test described herein. The thermoplastic composition is formed into a film to be used in the Water Absorption Test by melt coating the thermoplastic composition at a temperature of 180°C onto a release paper to obtain a film having the prescribed thickness of 200 µm. After cooling at room temperature the film is separated from the release paper.

A superabsorbent material in particle form available from Stockhausen GmbH & Co. KG under the code Z3049 is added to the thermoplastic polymeric composition while maintained at a temperature of 180°C and uniformly dispersed, in an amount corresponding to 42.9% by weight of the thermoplastic composition. The liquid absorbing thermoplastic material (Composition 2) has the following final composition by weight:

| | |
|---|---|
| 20% | Hytrel 8171 |
| 15% | PEG 400 |
| 34% | PEG 1500 |
| 30% | Z3049 |
| 1% | Irganox B 225 |

The mechanical strength in dry and wet state, in terms of tensile strength at break, and the breathability, in terms of Moisture Vapour Transmission Rate (MVTR), of the two thermoplastic absorbent materials of Examples 1 and 2 according to the present invention (Composition 1 and Composition 2) were measured according to the Tensile Strength Test and to the Moisture Vapour Transmission Test, respectively, both test methods described herein.

The Moisture Vapour Transmission Rate was measured on a film of the liquid absorbing thermoplastic material having a thickness of 200 µm. The film was made by melt coating the liquid absorbing thermoplastic material of Composition 1 and Composition 2 at a temperature of 180°C on a release paper to obtain a film having the desired thickness of 200 µm.

The results are shown in the table below, wherein T_{d} is the tensile strength in dry state, T_{w} is the tensile strength in wet state:

| | (T_{w}/T_{d})·100 | MVTR (g/m²·24h) |
|---|---|---|
| Composition 1 | >20% | >1000 |
| Composition 2 | >20% | >1000 |

### Examples 3 and 4

Liquid absorbent thermoplastic materials according to the present invention were also prepared (Compositions 3 and 4), having the same composition as Examples 1 and 2, respectively, but using a superabsorbent material in particle form available from Stockhausen Inc. USA under the name Cabloc 80HS. Tested according to the Tensile Strength Test, they also showed a tensile strength in wet state which is more than 20% of the tensile strength in dry state.

### TEST METHODS

### Water Absorption Test

The determination of the water absorption of pure thermoplastic polymers and of thermoplastic polymeric compositions, and of the liquid absorption of thermoplastic materials, is conducted according to the standard test method ASTM D 570-81 with the following conditions. The measurement of water absorption for pure thermoplastic polymers is made on the material in pellet form, with pellets having a diameter ranging from 3 mm to 5 mm, while the measurement of water absorption for thermoplastic polymeric compositions, and of liquid absorption for thermoplastic materials, is made on the material in form of a film sample 76.2 mm long by 25.4 mm wide by 0.2 mm thick.

For all materials a 24 hours immersion in liquid at 23°C was chosen and the percentage of liquid absorbed in accordance with the ASTM D 570-81 standard was reported. The liquid is distilled water for pure thermoplastic polymers and for thermoplastic polymeric compositions, and 0.9% NaCI water solution for thermoplastic materials comprising the matrix of thermoplastic polymeric composition with particles of superabsorbent material dispersed therein.

### Moisture Vapour Transmission Test

Breathability of the materials of the present invention is intended to be measured as Moisture Vapour Transmission Rate at 25°C and 55% relative humidity according to the modified ASTM E-96 "Upright Cup" method. The only modification to the standard ASTM E-96 "Upright Cup" method consists in a change in the height of the air gap between the sample and the water surface in the cup, which height is 4 mm ± 0.5 mm, instead of 19 mm ± 2.5 mm, as specified in the standard test method.

### Tensile Strength Test

The test measures the mechanical resistance of a sample of material as tensile strength at break, according to the standard test method ASTM D 412-92, under the following conditions. The test is performed on samples made of the liquid absorbing thermoplastic material and having a length of 130 mm, a width of 25.4 mm, and a thickness of 2 mm, and being continuous, obtained with any suitable method, for example by pouring the liquid absorbing thermoplastic material in molten state at a suitable temperature, e.g. 180°C for the materials of Example 1 and 2, into a metallic pan lined with release paper in a continuous layer having a thickness of 2 mm, and then after cooling cutting from this layer the samples of the desired dimensions.

The test is performed on samples made of the same composition both in dry and in wet state. In order to prepare the samples in wet state a sample is placed in a container of a saline solution (e.g. 0.9% NaCI water solution) maintained at a temperature of 23 ± 1°C, and shall rest entirely immersed for ten minutes. At the end of ten minutes, the sample shall be removed from the water, all surface water wiped off with a dry cloth, and tested for wet tensile strength as provided in the standard test method.

When starting from an article comprising the liquid absorbent thermoplastic material in turn comprising the matrix of thermoplastic polymeric composition with particles of water-insoluble water-swellable absorbent material dispersed therein, for example a disposable absorbent article with the thermoplastic material coated onto a substrate, the thermoplastic material can be isolated with known means in order to be tested. Typically in a disposable absorbent article the topsheet is removed from the backsheet and both are separated from any additional layers if present. The liquid absorbent thermoplastic material is scraped with a spatula from its substrate layer. The recovered thermoplastic material will be used to prepare samples as mentioned above with known means. For example, the thermoplastic material can be melted, or dissolved with a suitable solvent. Particles of superabsorbent material can be also separated from the thermoplastic material, in order to isolate the thermoplastic polymeric composition, as it is known in the art, for example by suitably sieving or filtering from the molten state, or preferably from the solution.

## Claims

1. A liquid absorbing thermoplastic material comprising a matrix of a thermoplastic polymeric composition having particles of water-insoluble water-swellable absorbent material dispersed therein, wherein said thermoplastic polymeric composition comprises:
a thermoplastic polymer or a mixture of thermoplastic polymers, and
a suitable compatible plasticiser or a blend of suitable compatible plasticisers,
**characterized in that** said thermoplastic polymeric composition has a water absorption greater than 30%, preferably greater than 40%, more preferably greater than 60%, most preferably greater than 90%, said water absorption measured according to the Water Absorption Test described herein.

2. A liquid absorbing thermoplastic material according to claim 1 **characterized in that** said material has a tensile strength in wet state which is at least 20%, preferably at least 40%, and more preferably at least 60%, of the tensile strength of said material in dry state, said tensile strengths evaluated according to the Tensile Strength Test described herein.

3. A liquid absorbing thermoplastic material comprising a matrix of a thermoplastic polymeric composition having particles of water-insoluble water-swellable absorbent material dispersed therein, wherein said thermoplastic polymeric composition comprises:
a thermoplastic polymer or a mixture of thermoplastic polymers, and
a suitable compatible plasticiser or a blend of suitable compatible plasticisers,
**characterized in that** said material has a tensile strength in wet state which is at least 20%, preferably at least 40%, and more preferably at least 60%, of the tensile strength of said material in dry state, said tensile strengths evaluated according to the Tensile Strength Test described herein.

4. A liquid absorbing thermoplastic material according to any preceding claim **characterized in that** said thermoplastic polymer or said mixture of thermoplastic polymers are selected from the group consisting of polyurethanes, poly-ether-amides block copolymers, polyethylene-acrylic acid and polyethylene-methacrylic acid copolymers, polyethylene oxide and its copolymers, ethylene acrylic esters and ethylene methacrylic esters copolymers, poly lactide and copolymers, polyamides, polyesters and copolyesters, polyester block copolymers, sulfonated polyesters, poly-ether-ester block copolymers, poly-ether-ester-amide block copolymers, polyacrylates, polyacrylic acids and derivatives, ionomers, polyethylene-vinyl acetate with a vinyl acetate content of at least 28% by weight, polyvinyl alcohol and its copolymers, polyvinyl ethers and their copolymers, poly-2-ethyl-oxazoline and derivatives, polyvinyl pyrrolidone and its copolymers, thermoplastic cellulose derivatives, poly-caprolactone and copolymers, poly glycolide, polyglycolic acid and copolymers, polylactic acid and copolymers, polyureas, and mixtures thereof, and wherein said thermoplastic polymer or each polymer of said mixture of thermoplastic polymers has a water absorption greater than 10%, preferably greater than 20%, more preferably greater than 30%, said water absorption measured according to the Water Absorption Test described herein.

5. A liquid absorbing thermoplastic material according to claim 4 **characterized in that** said thermoplastic polymer is selected from the group consisting of polyurethanes, copolyesters, polyester-amide block copolymers, polyether block copolymers, polyether-amide block copolymers, polyether-ester-amide block copolymers and polyether-ester block copolymers, and mixtures thereof.

6. A liquid absorbing thermoplastic material according to any preceding claim **characterized in that** said suitable compatible plasticiser or blend of suitable compatible plasticisers are selected from the group consisting of citric acid esters, tartaric acid esters, glycerol and its esters, sucrose esters, adipates, sebacates, sorbitol, epoxidized vegetal oils, polymerised vegetal oils, polyols, phthalates, liquid polyesters, glycolates, p-toluene sulfonamide and derivatives, glycols and polyglycols and their derivatives, sorbitan esters, phosphates, monocarboxylic fatty acids (C₈-C₂₂) and their derivatives, and mixtures thereof.

7. A liquid absorbing thermoplastic material according to claim 6 **characterized in that** said suitable compatible plasticiser or each plasticiser of said blend of suitable compatible plasticisers has a molecular weight (MW) greater than 300, preferably greater than 1000, more preferably greater than 3000.

8. A liquid absorbing thermoplastic material according to claim 7 **characterized in that** said suitable compatible plasticiser or each suitable compatible plasticiser of said blend of plasticisers is selected from the group consisting of glycerol esters, sucrose esters, sorbitol, epoxidized vegetal oils, polymerised vegetal oils, polyalcohols, polyols, liquid polyesters, p-toluene sulfonamide and derivatives, polyglycols and their derivatives, monocarboxylic fatty acids (C₈-C₂₂) and their derivatives, and mixtures thereof.

9. A liquid absorbing thermoplastic material according to any preceding claim **characterized in that** said matrix of thermoplastic polymeric composition comprises said thermoplastic polymer or mixture of polymers in an amount from 10% to 80%, preferably from 15% to 65%, and more preferably from 20% to 40% by weight of said matrix of thermoplastic polymeric composition, and comprises said suitable compatible plasticiser or blend of suitable compatible plasticisers in an amount from 20% to 90%, preferably from 35% to 85%, and more preferably from 60% to 80% by weight of said matrix of thermoplastic composition.

10. A liquid absorbing thermoplastic material according to any preceding claim **characterized in that** it comprises said water-insoluble water-swellable absorbent material in a amount from 10% to 90%, preferably from 15% to 70%, more preferably from 20% to 60% by weight of said liquid absorbing thermoplastic material.

11. A liquid absorbing thermoplastic material according to any preceding claim **characterized in that** said thermoplastic polymeric composition further comprises a tackifying resin or blend of tackifying resins.

12. A liquid absorbing thermoplastic material according to any preceding claim **characterized in that** said material has a liquid absorption of at least 5 g/g, preferably of at least 7 g/g, more preferably of at least 10 g/g, said liquid absorption measured according to the Water Absorption Test described herein.

13. A continuous layer formed from the liquid absorbing thermoplastic material according to any preceding claims, **characterized in that** said layer has a moisture vapour transmission rate (MVTR) of at least 300 g/m²·24h, more preferably of at least 500 g/m²·24h, even more preferably of at least 600 g/m²·24h, most preferably of at least 1000 g/m²·24h, with a thickness of said layer or film of at least 20 µm, said moisture vapour transmission rate measured according to the Moisture Vapour Transmission Test described herein.

14. An absorbent article comprising as a liquid absorbent material a liquid absorbing thermoplastic material according to any of the preceding claims.

15. An absorbent article comprising a liquid permeable topsheet, a liquid impermeable backsheet, preferably a breathable backsheet, and an absorbent core therebetween, said absorbent core comprising a liquid absorbing thermoplastic material according to any of the preceding claims 1 to 13.

16. An absorbent article according to claims 14 or 15, wherein said article is a disposable article for feminine protection, preferably a sanitary napkin or panty liner.
